# EUROPEAN PATENT SPECIFICATION

(11) **EP 2 197 392 B1**
(45) Date of publication and mention of the grant of the patent: **08.08.2018**
(21) Application number: 08799267.3
(22) Date of filing: 05.09.2008
(51) Int. Cl.: A61F 2/44, A61B 17/56

(54) **INTERSPINOUS SPACER**
INTERSPINÖSES ABSTANDSGLIED
ESPACEUR INTERÉPINEUX

(30) Priority: 07.09.2007 US 967805 P
(43) Date of publication of application: 23.06.2010
(73) Proprietor: Vertiflex, Inc., San Clemente, CA 92673 (US)
(72) Inventor: ALTARAC, Moti, Irvine, California 92603 (US); TEBBE, Shawn, Dromiskin, Co. Louth (IE); REGLOS, Joey Camia, Lake Forest, California 92630 (US); CHENG, Yang, Foothill Ranch, California 92610 (US)
(74) Representative: Donald, Jenny Susan
(86) International application number: PCT/US2008/075487
(87) International publication number: WO 2009/033093

(56) References cited:
- WO-A1-00/13619
- US-A1- 2003 065 330
- US-A1- 2006 264 938
- US-A1- 2007 106 385
- US-A1- 2007 185 490
- US-B2- 6 565 570

## Description

### FIELD

The present invention generally relates to medical devices, in particular, implants for placement between adjacent spinous processes of a patient's spine.

### BACKGROUND

With spinal stenosis, the spinal canal narrows and pinches the spinal cord and nerves, causing pain in the back and legs. Typically, with age, a person's ligaments may thicken, intervertebral discs may deteriorate and facet joints may break down-all contributing to the condition of the spine characterized by a narrowing of the spinal canal. Injury, heredity, arthritis, changes in blood flow and other causes may also contribute to spinal stenosis.

Doctors have been at the forefront with various treatments of the spine including medications, surgical techniques and implantable devices that alleviate and substantially reduce debilitating pain associated with the back. US2006264938 discloses an interspinous process implant having deployable wing. In one surgical technique, a spacer is implanted between adjacent spinous processes of a patient's spine. The implanted spacer opens the neural foramen, maintains the desired distance between vertebral body segments, and as a result, reduces impingement of nerves and relieves pain. For suitable candidates, an implantable interspinous spacer may provide significant benefits in terms of pain relief.

Any surgery is an ordeal. However, the type of device and how it is implanted has an impact. For example, one consideration when performing surgery to implant an interspinous spacer is the size of the incision that is required to allow introduction of the device. Small incisions and minimally invasive techniques are generally preferred as they affect less tissue and result in speedier recovery times. As such, there is a need for interspinous spacers that work well with surgical techniques that are minimally invasive and provide quick, easy and effective solutions for doctors and their patients. The present invention sets forth such a spacer.

### SUMMARY

According to one aspect of the invention, an implantable spacer for placement between adjacent spinous processes is provided. The adjacent spinous processes includes a superior spinous process and an inferior spinous process. Each of the superior and inferior spinous processes has two lateral surfaces. The implantable spacer includes a body having a longitudinal axis. A wing is connected to the body and capable of movement with respect to the body. The wing has at least a first pair of extension members having longitudinal axes. The wing has at least one caming surface. The spacer further includes an actuator assembly connected to the body. The actuator assembly includes an actuator and a shaft connected to the actuator. The actuator assembly is configured such that the actuator is disposed inside the body such that the shaft is accessible at the proximal end of the spacer. The actuator is configured to move relative to the spacer body to contact the caming surface of the wing to move the wing from a first position to a second position.

According to another aspect of the invention, an implantable spacer for placement into an interspinous process space between adjacent spinous processes is provided. The adjacent spinous processes include a superior spinous process and an inferior spinous process. The implantable spacer includes a body having longitudinal axis, a first end and a second end. The first end is configured to be positioned inside the interspinous process space proximally to the spinal canal relative to the second end. The spacer further includes at least one movable element and a mechanism for moving the at least one movable element from a first position to a second position. The at least one movable element is configured to laterally stabilize the spacer relative to at least one of the superior or inferior spinous process when in said second position. The mechanism is configured such that movement of the at least one movable element from the first position to the second position is effected by moving the mechanism relative to the spacer body in a direction away from spinal canal.

According to another aspect of the invention, an implantable spacer for placement into an interspinous process space between adjacent spinous processes is provided. The adjacent spinous processes include a superior spinous process and an inferior spinous process. The implantable spacer includes a body having longitudinal axis, a first end and a second end. The first end is configured to be positioned inside the interspinous process space proximally to the spinal canal relative to the second end. The spacer further includes at least one movable element. The spacer also includes an actuator assembly connected to the body. The actuator assembly includes an actuator mechanism for moving the at least one element from a first position to a second position. The at least one movable element is configured to laterally stabilize the spacer relative to at least one of the superior or inferior spinous processes when in said second position. The spacer includes a locking mechanism for locking the at least one movable element in said second position. The locking mechanism includes a body link having at least one outer surface angled with respect to the longitudinal axis and configured such that effecting movement of the at least one element from a first position to a second position moves the body link relative to the body to create a force to lock the at least one movable element in place.

According to another aspect of the invention, an implantable spacer for placement into an interspinous process space between adjacent spinous processes is provided. The adjacent spinous processes include a superior spinous process and an inferior spinous process. The implantable spacer includes a spacer body and movable wing combination. The movable wing has a first position and a second position and at least one extension member for laterally stabilizing the spacer body with respect to the at least one spinous process when in said second position. The at least one extension member shares the length of the spacer body when in said first position.

According to another aspect of the invention, an implantable spacer for placement into an interspinous process space between adjacent spinous processes is provided. The adjacent spinous processes include a superior spinous process and an inferior spinous process. The implantable spacer includes a body having longitudinal axis, a first end and a second end. The body has a superior spinous process engaging surface and an inferior spinous process engaging surface. The spacer includes at least one movable element and an actuator assembly. The actuator assembly is connected to the body and configured for moving the at least one movable element from a first position to a second position. The at least one movable element is configured to laterally stabilize the spacer relative to at least one of the superior or inferior spinous processes when in said second position. When in the second position, the spacer is positionable within the interspinous process space such that the superior spinous process engaging surface faces the superior spinous process and the inferior spinous process engaging surface faces the inferior spinous process. The spacer is configured to abut at least one of the superior spinous process and inferior spinous process on a corresponding superior spinous process engaging surface and inferior spinous process engaging surface at a location along the body that is outside the location of the movable element when in the second position.

### BRIEF DESCRIPTION OF THE DRAWINGS

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity.
FIG. 1a illustrates a perspective view of a spacer in an undeployed configuration according to the present invention.
FIG. 1b illustrates a perspective view of a spacer in a deployed configuration according to the present invention.
FIG. 2 illustrates an exploded perspective view of a spacer according to the present invention.
FIG. 3a illustrates a perspective view of a body of a spacer according to the present invention.
FIG. 3b illustrates a side view of a body of a spacer according to the present invention.
FIG. 3c illustrates a top view of a body of a spacer according to the present invention.
FIG. 3d illustrates a cross-sectional view taken along line F-F in FIG. 3c of a body of a spacer according to the present invention.
FIG. 4a illustrates a perspective view of a wing according to the present invention.
FIG. 4b illustrates a top view of a wing according to the present invention.
FIG. 4c illustrates a side view of a wing according to the present invention.
FIG. 4d illustrates a cross-sectional view taken along line J-J in FIG. 4b of a body of a spacer according to the present invention.
FIG. 4e illustrates a cross-sectional view taken along line H-H in FIG. 4b of a body of a spacer according to the present invention.
FIG. 5a illustrates a perspective view of an actuator of a spacer according to the present invention.
FIG. 5b illustrates a side view of an actuator of a spacer according to the present invention.
FIG. 6a illustrates a perspective view of a shaft of a spacer according to the present invention.
FIG. 6b illustrates a side view of a shaft of a spacer according to the present invention.
FIG. 7a illustrates a perspective view of a body link of a spacer according to the present invention.
FIG. 7b illustrates a cross-sectional view of a body link of a spacer according to the present invention.
FIG. 8 illustrates a cross-sectional view of a spacer in an undeployed configuration according to the present invention.
FIG. 9 illustrates a cross-sectional view of a spacer in a deployed configuration according to the present invention.
FIG. 10 illustrates a spacer according to the present invention deployed in an interpsinous process space between two adjacent vertebral bodies and a supraspinous ligament.

### DETAILED DESCRIPTION

Before the subject devices, systems are described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs.

It must be noted that as used herein and in the appended claims, the singular forms "a", "an", and "the" include plural referents unless the context clearly dictates otherwise. Thus, for example, reference to "a spinal segment" may include a plurality of such spinal segments and reference to "the screw" includes reference to one or more screws and equivalents thereof known to those skilled in the art, and so forth.

The present invention is described in the accompanying figures and text as understood by a person having ordinary skill in the field of spinal implants and implant delivery instrumentation.

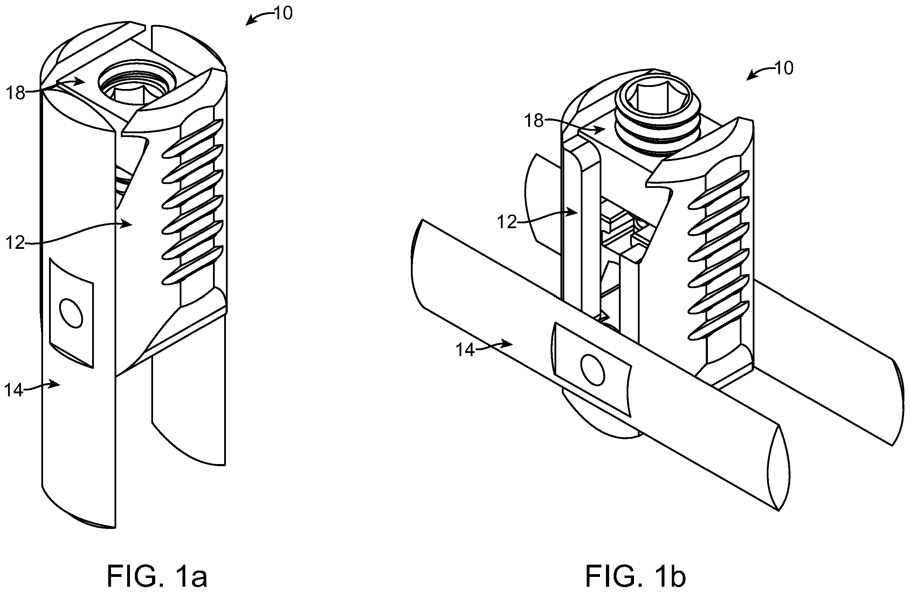

With reference to FIGs. 1a and 1b, a spacer 10 according to the present invention is shown. FIG. 1a illustrates the spacer 10 in a first position or undeployed configuration and FIG. 1b illustrates the spacer 10 in a second position or deployed configuration. The spacer 10 includes a body 12, an extension member, wing or arm 14, and an actuator assembly 18. The wing 14 and the actuator assembly 18 are connected to the body 12. When in the undeployed configuration shown in FIG. 1a, the longitudinal axis of the wing 14 is substantially parallel to the longitudinal axis of the body 12 whereas when in the deployed configuration shown in FIG. 1b, the wing 14 is substantially perpendicular to the longitudinal axis of the body 12. As seen in FIG. 1a, a portion of the wing 14 overlaps or shares a length of the body 12, thereby, advantageously reducing the length of the overall spacer 10.

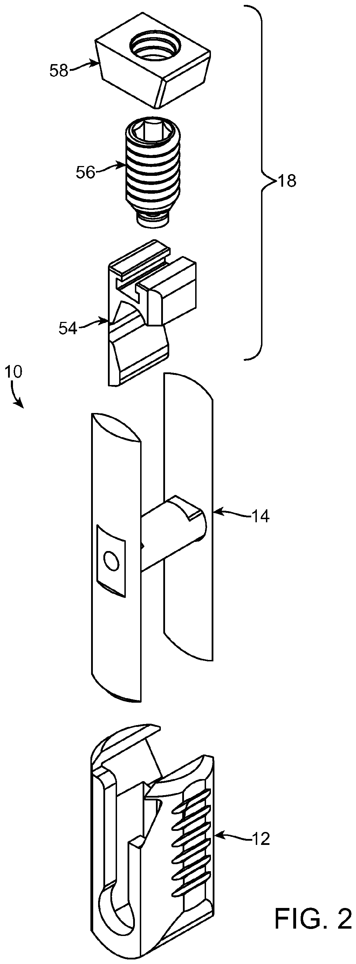

Turning to FIG. 2, an exploded perspective view of the spacer 10 is shown illustrating the body 12, wing 14 and components of the actuator assembly 18.

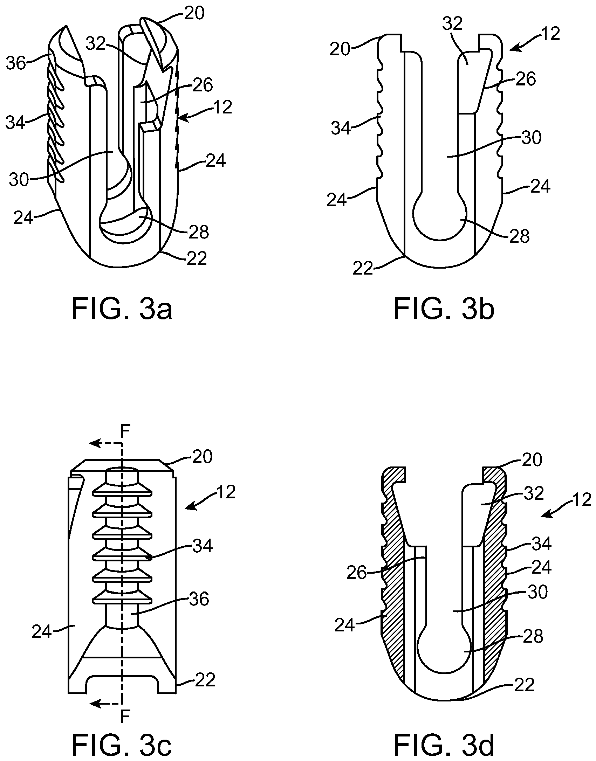

Turning to FIGs. 3a, 3b, 3c and 3d, there is shown a perspective view, side view, top view and sectional view, respectively, of the body 12 according to the present invention. The body 12 has a size and shape that allows for implantation between adjacent spinous processes and facilitates delivery into a patient through a narrow port or cannula. The body 12 has a proximal end 20 and a distal end 22 and two oppositely located sidewalls 24 integrally joined at the distal end 22. When implanted in an interspinous process space, one of the sidewalls 24 serves as a superior spinous process engaging surface and the other serves as an inferior spinous process engaging surface. In one variation, the sidewalls 24 are substantially flat surfaces and substantially parallel to each other. The body 12 forms a generally U-shaped channel between the sidewalls 24 with the open end of the U-shaped channel located at the proximal end 20. Inside the body 12, the body 12 defines an actuator assembly receiving portion 26 and a wing receiving portion 28 between the sidewalls 24. The wing receiving portion 28 is located near the distal end 22 of the body 12 and is connected to the actuator assembly receiving portion 26 which together form the U-shaped passageway 30 inside the body 12. The wing receiving portion 28 is arcuate in shape which provides the wing 14 with a smooth bearing surface for rotation. The actuator assembly receiving portion 26 includes a body link receiving portion 32.

The outside of the body 12 includes ridges 34 along at least a portion of the sidewalls 24. In one variation, the body 12 does not include ridges 34. The ridges 34 and sidewalls 24 on which they are formed function to provide a traction surface for contact with the ends of the spinous processes of the superior and inferior vertebrae or other tissue of the interspinous process space between which the spacer 10 is implanted. When implanted, one of the sidewalls 24 faces the superior spinous process and the other sidewall 24 faces the inferior spinous process. The distance between sidewalls is sufficient to occupy the interspinous process space according to surgeon preference. In one variation, the ridges 34 are angled towards the proximal end 20 to ease insertion and help prevent the spacer from backing out as the ridges grip the spinous processes and adjacent tissue to help keep the spacer 10 in place. In one variation, as shown in FIG. 3c, a slight saddle-shaped channel or scallop 36 is formed on the outer surface of the sidewalls 24 extending longitudinally between the proximal end 20 and the distal end 22 to help seat, conform and center the body 12 between spinous processes. The channel 36 is shown in conjunction with ridges 34 in FIGs. 3a and 3c. The distal tip 22 of the spacer body 12 is rounded to ease passage of the spacer 10 through tissue and ligament. The distal tip 22 serves as the leading end of the spacer 10 being positionable closer to the spinal canal relative to the proximal end 20.

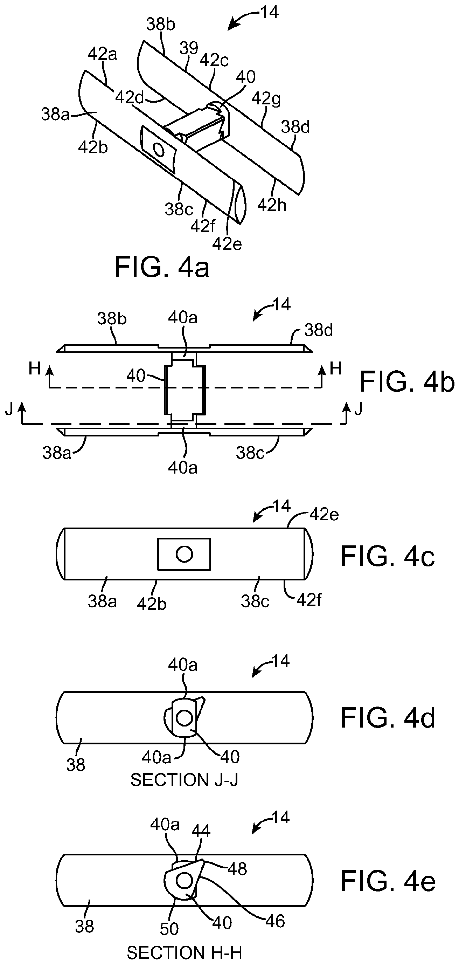

With reference now to FIGs. 4a-4e, there is shown a perspective, top, side, a first-cross-sectional and a second cross-section view, respectively, of the wing 14 according to the present invention. The wing 14 includes at least two extending members 38a, 38b interconnected by a cross-member 40 that together form a single U-shaped channel. In the variation shown in FIGs. 4a-4e, four extending members 38a, 38b, 38c and 38d are part of the spacer 10. The four extending members 38a, 38b, 38c and 38d are interconnected by at least one cross-member 40 and form two adjacent generally U-shaped channels such that together, the U-shaped channels form a generally H-shaped wing 14 as seen in FIG. 4b. One substantially U-shaped channel is defined between extending members 38a and 38b configured and sized for receiving a superior spinous process and laterally retaining the spacer with respect to the superior spinous process and a second substantially U-shaped channel is defined between extending members 38c and 38d configured and sized for receiving an inferior spinous process and laterally retaining the spacer with respect to the inferior spinous process. The inner surfaces of the extending members may contact or engage or conform to and generally face the lateral sides of the spinous processes when the spacer is implanted. In this regard, the extending members are configured and dimensioned to generally prevent or limit lateral movement of the spacer when the spacer is implanted. In the variation shown, extending members 38a and 38c form one side of the wing 14 and have longitudinal axes that are coincident. Also, extending members 38b and 38d form a second side of the wing 14 and have longitudinal axes that are coincident. Each extending member 38a, 38b, 38c, 38d is substantially rectangular in shape. In another variation, the extending is any suitable shape for preventing or limiting lateral movement of the spacer with respect to at least one of the spinous processes. Each extending member 38a-38d includes a substantially flat inner surface and a slightly curved outer surface. The curved outer surface contributes to the bullet-like profile of the spacer 10 when in the undeployed configuration and conforms more closely to the shape of the body 12 to ease installation as the spacer is moved through tissue to the interspinous process space. The flat inner surface and the curved outer surface of each extending member 38 meet to form edges 42a, 42b, 42c, 42d. In one variation, the edges 42a, 42b, 42c, 42d are relatively sharp and therefore, advantageous for passing or cutting through tissue as the wing 14 is moved from an undeployed configuration to a deployed configuration.

With particular reference to FIG. 4e, the cross-member 40 includes a first caming surface 44 and a second caming surface 46. The first and second caming surfaces 44, 46 are angled with respect to each other to form a wedge-shapesuch that one end forms a pointed lock engaging end 48 for engaging with the actuator and the other end forms a curved seating end 50 for seating in the wing receiving portion 28 of the body 12. The cross-member 40 includes end portions 40a configured as curved seating surfaces for seating in the wing receiving portion 28 of the body 12. The curved seating surfaces extend around at least half of the circumference of the cross-member 40. The cross-member 40 is fixed with respect to the extending members 38a, 38b, 38c, 38d such that movement of the cross-member 40 translates to movement of the extending members 38a, 38b, 38c, 38d.

With brief reference back to FIG. 2, the actuator assembly 18 will now be described. The actuator assembly 18 includes an actuator 54, a shaft 56 and an optional body link 58. The body link 58 and actuator 54 are connected to the shaft 56.

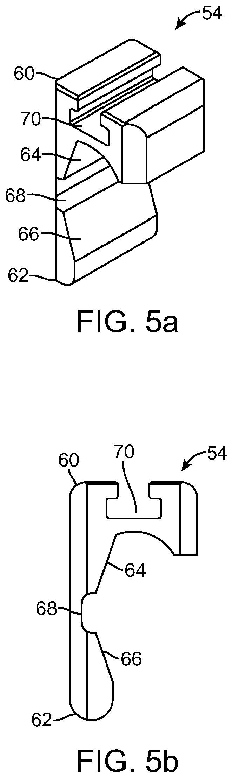

Turning now to FIGs. 5a and 5b, the actuator 54 will now be described. The actuator 54 includes a proximal end 60 and a distal end 62, a first surface 64, a second surface 66, a receiving portion 68 for the pointed lock engaging end 48 of the cross member 40 and a shaft receiving portion 70 configured to receive the shaft 56. The first surface 64 is configured to conform and correspond to the first caming surface 44 and curved seating end 50 of the cross member 40 when the spacer 10 is in the undeployed configuration such that the first caming surface 44 and curved seating end 50 of the cross member 40 are in juxtaposition with the first surface 64 of the actuator 54. The second surface 66 is configured to conform and correspond to the second caming surface 46 of the cross member 40 when the spacer is in the deployed configuration. The first surface 64 and the second surface 66 define a wedge-shaped space for receiving the cross-member 40. The receiving portion 68 is configured to receive and retain the pointed lock engaging end 48 of the cross member 40. First and second surfaces 64 and 66 are configured to be substantially at the same angle with respect to the longitudinal axis permitting rotation of the cross-member by approximately 90 degrees. The first and second surfaces 64 and 66 in conjunction with the receiving portion 68 serve as bearing surfaces for the first and second caming surfaces 44, 46 to effect rotation of the wing 14 to and from an undeployed configuration and a deployed configuration. In one variation, the first surface 64 bears at least part of the force from the first caming surface 44 for moving the wing 14 from a first position to a second position and the second surface 66 bears at least part of the force from the second caming surface 46 when the wing is in the second position preventing the wing from over-rotation. The distal end 62 of the actuator 54 is bulbous and configured to retain the cross member 40 within the actuator 54 when the spacer 10 is assembled.

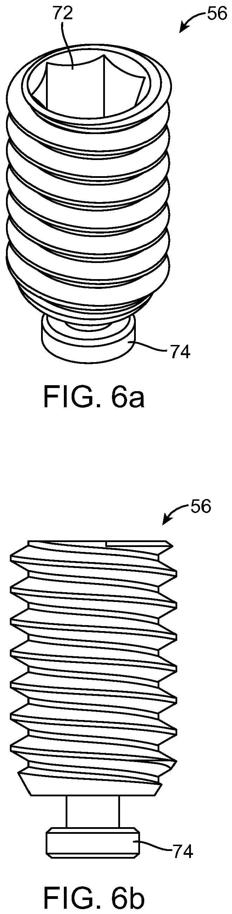

Turning now to FIGs. 6a and 6b, the shaft 56 of the actuator assembly 18 will now be described. The shaft 56 is substantially cylindrical in shape and, in one variation, includes a threaded outer surface for engagement with the threaded inner surface of the body link 58. In a variation without a body link 58, the threaded outer surface of the shaft 56 engages with a threaded inner surface of the body 12. The proximal end of the shaft 56 includes a socket 72 such as a hex socket for receiving a hexagonally-shaped driving tool. When the spacer 10 is assembled, the proximal end of the shaft 56 is accessible at the proximal end of the spacer 10 for connection with a driving tool. The distal end of the shaft 56 includes an actuator engagement portion 74 configured to connect to the actuator 54. The actuator engagement portion 74 is a projection that connects to the shaft receiving portion 70 on the actuator 54.

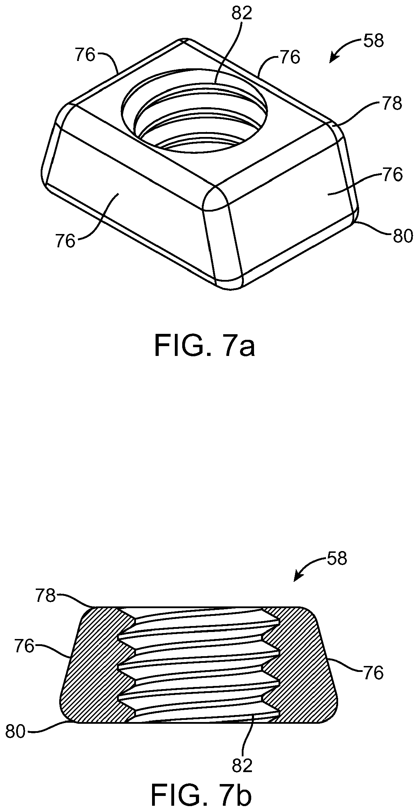

Turning now to FIGs. 7a and 7b, the body link 58 will now be described. The body link 58 is sized and configured to be disposed inside the link receiving portion 32 of the body 12 and configured to link the shaft 56 to the body 12. The body link 58 includes a threaded bore 82 configured to receive the threaded shaft 56. In the variation of FIGs. 7a and 7b, the body link 58 further functions as a body expander such that the body link 58 includes at least one diverging outer surface 76. The at least one angled surface is configured such that it diverges from proximal end 78 toward the distal end 80 of the body link 58. As a result, the body link 58 is larger at the distal end 80 relative to the proximal end 78. In the variation shown in FIGs. 7a and 7b, the angled outer surface 76 comprises four angled sides which in combination diverge outwardly from the proximal end 78 toward the distal end 80 to form a wedge-like shape. However, the invention is not so limited so long as the body link 58 has a diverging surface. Another example of a diverging body link 58 is a body link 58 having a cone-shaped outer surface. Whether the variation of the spacer includes a diverging or non-diverging body link 50, the shape of the link receiving portion 32 corresponds to the shape of the body link 50 and the link receiving portion 32 is sufficiently large enough to permit the body link 50 to travel inside it as the shaft 56 is moved to deploy the wing 14.

Assembly of the actuator assembly 18 will now be described in reference to FIGs. 2, 5a, 5b, 6a, 6b, 7a and 7b. The shaft 56 of the actuator assembly 18 is connected to the actuator 54 by inserting the actuator engagement portion 74 of the shaft 56 into the shaft receiving portion 70 of actuator 54. The shaft receiving portion 70 is a slot with a constricted neck portion into which the actuator engagement portion 74 of the shaft 56 slides laterally into and cannot be removed along the longitudinal axis. The shaft 56 is connected to the body link 58 by inserting the threaded portion of the shaft 56 into the threaded bore 82 of the body link 58 to complete the assembly of the actuator assembly 18.

Assembly of the remainder of the spacer 10 will now be described. The wing 14 is connected to the actuator assembly 18. The wing 14 is connected to the actuator 54 such that the pointed lock engaging end 48 of the cross member 40 of the wing 14 is inserted into the receiving portion 68 of the actuator 54. The wing 14 and actuator assembly 18 are inserted through the opening at the proximal end 20 of the body 12 until the wing 14 is seated in the wing receiving portion 28, the actuator assembly 18 is disposed inside the actuator assembly receiving portion 26 and the body link 58 is located in the body link receiving portion 32. The end portions 40a of the cross-member 40 rest against corresponding curved surfaces of the wing receiving portion 28 of the body 12 advantageously providing a large contact surface area suitable for bearing large loads, in particular, shear forces on the wing. The body link 58 is inserted and snapped through the opening at the proximal end 20 of the body 12 into the complementarily-shaped body link receiving portion 32 and retained therein via an interference fit engagement with the body 12. With the body link 58 in place, the wing 14 and the actuator assembly 18 are secured inside the body 12. The wing 14 is seated in wing receiving portion 28 such that wing 14 is capable of rotational movement with respect to the body 12.

Once assembled, the spacer 10 is ready for delivery into the patient. To deliver the spacer 10 within the patient, the spacer 10 is releasably attached to a delivery instrument (not shown). For example, a delivery instrument may connect to the proximal end 20 of the spacer 10 via notches (not shown) formed in the body 12 or connect to outer holes (not shown) formed in the cross member 40 of the wing 14. The spacer 10 is provided or otherwise placed in its undeployed state or closed configuration as illustrated in FIG. 1a wherein at least a part of the length of the wing 14 shares/overlaps a part of the length of the body 12 when in an undeployed configuration and, in particular, at least half of the length of the wing 14 is shared/overlapped by the length of the body 12. A small midline or lateral-to-midline posterior incision is made in the patient for minimally-invasive percutaneous delivery. In one variation, the supraspinous ligament is avoided. In another variation, the supraspinous ligament is split longitudinally along the direction of the tissue fibers to create an opening for the instrument. Dilators may be further employed to create the opening. In the undeployed state and attached to a delivery instrument, the spacer 10 is inserted through a port or cannula, if one is employed, which has been operatively positioned to an interspinous process space within a patient's back with the proximal end extending outside the patient. In some circumstances, it may not be necessary to use a cannula where the device is inserted with the delivery instrument alone or through a larger opening in the tissue. The spacer is then advanced to within the targeted interspinous process space between two adjacent spinous processes. If a cannula is employed, the spacer 10 is advanced beyond the end of the cannula or, alternatively, the cannula is pulled proximately to uncover the spacer 10 within. The surgeon may examine the positioning of the spacer 10 via fluoroscopy and reposition it if necessary.

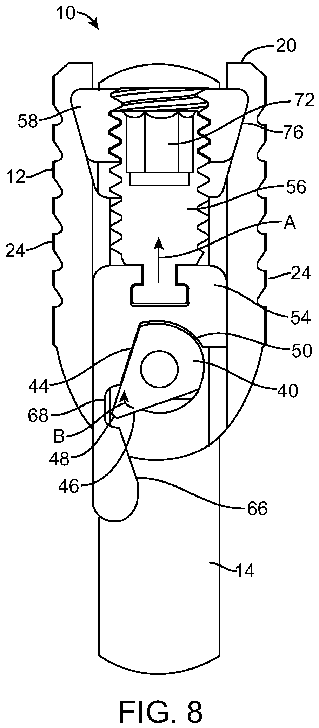

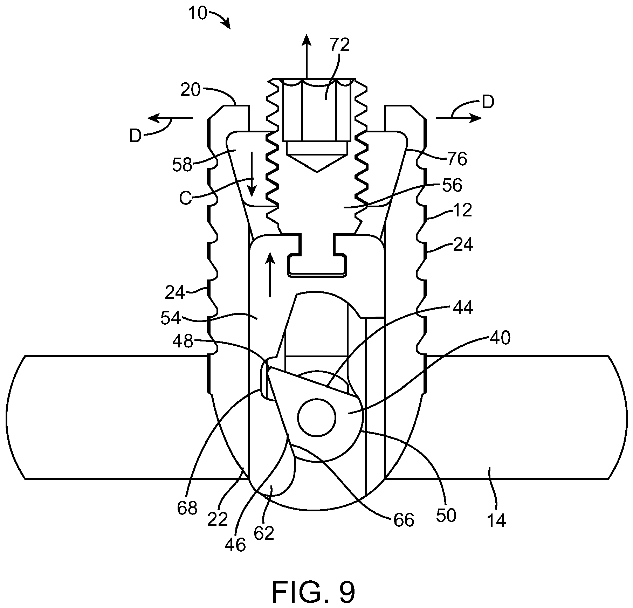

With particular reference now to FIGs. 8 and 9, deployment of the spacer 10 from an undeployed configuration illustrated in FIG. 8 to a deployed configuration illustrated in FIG. 9 while positioned within the interspinous process space will now be described. With particular reference first to FIG. 8, a driver (not shown) such as a hex-shaped tool is inserted into the hex socket 72 of the shaft 56 and turned to move or pull the shaft 56 towards the proximal end 20 of the body 12 in a direction indicated by the arrow "A". Since the actuator 54 is connected to the shaft 56, the actuator 54 also moves (is pulled) towards the proximal end 20 rotating the wing 14 in a direction indicated by the arrow "B". The entire wing 14 rotates through an angle of approximately 90 degrees from the undeployed configuration through intermediate configurations into the second or deployed configuration shown in FIG. 9 in which the wing 14 is perpendicular to the longitudinal length of the body 12. The proximal direction of motion of the shaft 56 and connected actuator 54 relative to the body 12 (pull deployment) advantageously avoids pushing the spacer 10 deeper into the interspinous space and towards the spinal canal during the process of deployment. Instead, the proximal direction of motion or pulling of the actuator assembly 18 provides for a safer implant and a secure positioning easing installation for the surgeon. The surgeon may examine the positioning of the spacer 10 via fluoroscopy with the spacer 10 in an intermediate configuration and choose to reposition it by moving the spacer 10 along a general posterior-anterior direction with the wings 14 partially deployed. Alternatively, the surgeon may choose to reposition the spacer 10 by returning the spacer 10 to first or closed configuration by rotating the driver in an opposite direction and then moving the spacer 10 into position and continuing with deployment of the wings 14.

With particular reference to FIG. 9, in the deployed configuration the second surface 66 of the actuator 54 abuts the second caming surface 46 of the cross member 40. Further rotation of the wing 14 is prevented by the bulbous distal end 62 being lodged or wedged between the cross member 40 and distal end 22 of the body 12. If the shaft 56 is further proximally advanced pulling the actuator 54 proximally along with it, the wing 14 will not rotate any further; however, in a variation of the spacer 10 that includes a body link 58 that functions as an expander as described above, the body link 58 will advance distally in a direction indicated by arrow "C" in FIG. 9. The diverging outer surface 76 of the body link 58 will wedge toward the distal end 22 spreading the proximal end 20 of the sidewalls 24 outwardly in a direction indicated by arrows "D" relative to the distal end of the sidewalls 24. The spring force of the outwardly biased sidewalls 24 will exert a force from both directions back onto the shaft 56 tightening it in place, thereby, advantageously providing a self locking feature that prevents the threaded shaft or screw 56 from backing out and the wing collapsing. Also, the expanded proximal end 20 of the sidewalls 24 provides additional customized distraction of the spinous processes. The surgeon can drive the shaft 56 to further spread the sidewalls 24 thereby providing greater distraction of the spinous processes according to surgeon preference giving the surgeon additional flexibility in selecting the degree of distraction for a particular patient. Furthermore, the outwardly expanded proximal end 20 of the sidewalls 24 creates a wedge-shaped seat for the spinous process. With the sidewalls 24 in an expanded configuration, the spacer 10 assumes an overall wedge-like shape advantageous for retainment in the interspinous process space. With the sidewalls 24 in an expanded configuration the wedge-shaped seat forms an angle between the sidewall 24 and the wing 14 that is slightly less than 90 degrees on each side of the body 12. This feature advantageously secures the spacer 10 within the patient and helps keep it in place between spinous processes.

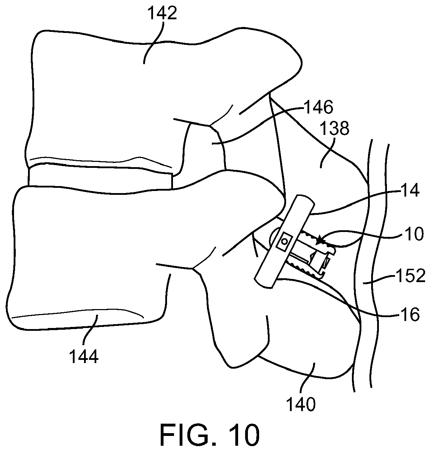

The spacer 10 may be undeployed for removal from the interspinous space by rotating the shaft 56 in the opposite direction to fold the wing 14 into the closed or undeployed configuration or any intermediate configuration. In the undeployed configuration, the spacer 10 can be removed from the patient or re-adjusted and re-positioned and then re-deployed as needed. This process can be repeated as necessary until the clinician has achieved the desired positioning of the spacer in the patient. Following final positioning, the driver and delivery instrument is detached from the spacer 10 and removed from the operative site leaving the spacer 10 implanted in the interspinous process space as shown in FIG. 10. In FIG. 10, the spacer 10 is shown with the wing 14 receiving the superior spinous process 138 of a first vertebral body 142 and the inferior spinous process 140 of an adjacent second vertebral body 144 providing sufficient distraction/spacing to open the neural foramen 146 to relieve pain. In one variation of the spacer 10 of the present invention, the spacer 10 is configured such that the body 12 seats the superior and inferior spinous processes 138, 140 at a location along the length of the body 12 that is outside location of the wing 14 when in the deployed configuration. Hence, the wing 14 serves as a lateral stabilizer, locator for the spacer 10 instead of a seating location for the spinous processes 138, 140. Therefore, the spacer 10 provides for a longer seating location for the superior and inferior spinous processes making it easier for the surgeon to install the spacer 10. In one variation, the shape of the arm 14 is such that it conforms to the spinous processes 138, 140. The supraspinous ligament 152 is also shown in FIG. 10. The spacer 10 maintains the spinous processes in a distracted or spaced condition, for example where the distance of the implant is greater than a pre-implantation distance between the spinous processes.

The wing 14 is movably or rotatably connected to the body 12 to provide rotational movement from an undeployed configuration to a deployed configuration that arcs through about a 90 degree range or more. The wing 14 is rotationally movable between at least an undeployed, collapsed or folded state (as shown in FIG. 8) and a fully deployed state (as shown in FIG. 9). In the undeployed state, the wing 14 is aligned generally or substantially axially (i.e., axially with the longitudinal axis defined by the body 12 or to the translation path into the interspinous process space of the patient) to provide a minimal lateral or radial profile. In the deployed state, the wing 14 is positioned generally or substantially transverse to the collapsed position (i.e., transverse to the longitudinal axis defined by the body 12 or to the translation path into the interspinous space of the patient). In another variation, the wing 14 may also be linearly moveable or translatable from the deployed state to and from an additionally extended state. More specifically, the wing 14 can be extended in the general vertical or horizontal direction along an axis substantially parallel or perpendicular to the spine. The wing 14 is connected to the body 12 in a manner that enables it to be moved simultaneously or independently of each other, as well as in a manner that provides passive deployment and/or vertical extension or, alternatively, active or actuated deployment and/or vertical extension.

The spacer 10 is as easily and quickly removed from the body of the patient as it is installed. To remove the spacer 10, the delivery instrument is inserted into an incision and reconnected to the spacer 10. The shaft 56 is rotated in the opposite direction via a driver to fold the wing 14 into a closed or undeployed configuration such that the wing 10 is clear or disengaged from the superior and inferior spinous processes. In the undeployed configuration, the spacer 10 can be removed from the patient along with the instrument or, of course, re-adjusted and re-positioned and then re-deployed as needed with the benefit of minimal invasiveness to the patient.

Any of the spacers disclosed herein are configured for implantation employing minimally invasive techniques including through a small percutaneous incision and through the superspinous ligament. Implantation through the superspinous ligament involves selective dissection of the superspinous ligament in which the fibers of the ligament are separated or spread apart from each other in a manner to maintain as much of the ligament intact as possible. This approach avoids crosswise dissection or cutting of the ligament and thereby reduces the healing time and minimizes the amount of instability to the affected spinal segment. While this approach is ideally suited to be performed through a posterior or midline incision, the approach may also be performed through one or more incisions made laterally of the spine with or without affect to the superspinous ligament. Of course, the spacer may also be implanted in a lateral approach that circumvents the superspinous ligament altogether.

Other variations and features of the various mechanical spacers are covered by the present invention. For example, a spacer may include only a single U-shaped arm which is configured to receive either the superior spinous process or the inferior spinous process. The surface of the spacer body opposite the side of the single arm may be contoured or otherwise configured to engage the opposing spinous process wherein the spacer is sized to be securely positioned in the interspinous space and provide the desired distraction of the spinous processes defining such space.

Furthermore, depending on the variation of the spacer employed, distraction of the interspinous space is provided by the body of the spacer such that the superior and inferior spinous processes rest on either side of the body and the H-shaped wing keeps the spacer in position with each U of the H-shaped wing encompassing at least a portion of the spinous process. Alternatively, distraction of the interspinous process space is provided by the wing such that each U of the H-shaped wing supports the superior and inferior spinous processes within the U-shaped saddle. The U-shaped saddle can be made shallower or deeper to provide a desired amount of distraction for the spinous processes.

The extension arms of the subject device may be configured to be selectively movable subsequent to implantation, either to a fixed position prior to closure of the access site or otherwise enabled or allowed to move in response to normal spinal motion exerted on the device after deployment. The deployment angles of the extension arms may range from less than 90 degrees (relative to the longitudinal axis defined by the device body) or may extend beyond 90 degrees. Each extension member may be rotationally movable within a range that is different from that of the other extension members. Additionally, the individual superior and/or inferior extensions may be movable in any direction relative to the strut or bridge extending between an arm pair or relative to the device body in order to provide shock absorption and/or function as a motion limiter, or serve as a lateral adjustment particularly during lateral bending and axial rotation of the spine. The manner of attachment or affixation of the extensions to the arms may be selected so as to provide movement of the extensions that is passive or active or both. In one variation, the saddle or distance between extensions can be made wider to assist in seating the spinous process and then narrowed to secure the spinous process positioned between extensions.

The disclosed devices or any of their components can be made of any biologically adaptable or compatible materials. Materials considered acceptable for biological implantation are well known and include, but are not limited to, stainless steel, titanium, tantalum, combination metallic alloys, various plastics, polymers, resins, ceramics, biologically absorbable materials and the like. Polymers including PEEK, PEK, PAEK, PEKEKK or any polyetherketone or polyetherketone metal composite can be employed. In the variation in which the body link 58 is configured as an expander, a slightly flexible construction of the body 12 is desirable to effect the desired self-locking features described above in which case suitable materials such as polymeric materials are appropriately selected for the entire spacer or for selected components of the spacer. Any component may be also coated/made with osteo-conductive (such as deminerized bone matrix, hydroxyapatite, and the like) and/or osteo-inductive (such as Transforming Growth Factor "TGF-B," Platelet-Derived Growth Factor "PDGF," Bone-Morphogenic Protein "BMP," and the like) bio-active materials that promote bone formation. Further, a surface of any of the implants may be made with a porous ingrowth surface (such as titanium wire mesh, plasma-sprayed titanium, tantalum, porous CoCr, and the like), provided with a bioactive coating, made using tantalum, and/or helical rosette carbon nanotubes (or other carbon nanotube-based coating) in order to promote bone ingrowth or establish a mineralized connection between the bone and the implant, and reduce the likelihood of implant loosening. Lastly, any assembly or its components can also be entirely or partially made of a shape memory material or other deformable material.

## Claims

1. An implantable spacer (10) for placement between adjacent spinous processes, the adjacent spinous processes including a superior spinous process and an inferior spinous process, each of the superior and inferior spinous processes having two lateral surfaces, the implantable spacer comprising:
a body (12) having a longitudinal axis, a first end (22) and a second end (20);
the first end (22) configured to be positioned inside the interspinous process space proximally to the spinal canal relative to the second end (20);
a wing (14) connected to the body (12) and capable of movement with respect to the body (12);
the wing (14) having at least one caming surface (44, 46);
an actuator assembly (18) connected to the body (12);
the actuator assembly (18) comprising:
an actuator (54); and
a shaft (56) connected to the actuator (54);
the actuator assembly (18) is configured such that the shaft (56) is accessible at the proximal end of the spacer(20); and
the actuator (54) is configured to move relative to the body (12) to contact the caming surface (44; 460 of the wing to move the wing (14) from a first position to a second position for laterally stabilizing the spacer (10) relative to at least one of the superior or inferior spinous process when in said second position,
**characterised in that** the body (12) comprises a first lateral side (24), and a second lateral side (24);
the wing (14) comprises a first pair of extension members (38a, 38c) having first longitudinal axes, at the first lateral side (24) of the body (12), and a second pair of extension members (38b, 38d) having second longitudinal axes, at the second lateral side (24) of the body(12);
wherein the wing (14) is capable of rotational movement with respect to the body (12) to rotate the wing (14) from the first to the second position;
wherein the first longitudinal axes of the first pair of extension members (38a, 38c) are coincident with each other in the first and second positions, and the second longitudinal axes of the second pair of extension members (38b, 38d) are coincident with each other in the first and second positions.

2. The spacer (10) of claim 1 wherein each extension member has (38a, 38b, 38c, 38d) an inner surface and an outer surface and when in the second position, in use, each inner surface of each extension member (38a, 38b, 38c, 38d) faces at least one or more lateral surface of a spinous process.

3. The spacer (10) of claim 2 wherein the inner surface of each extension member(38a, 38b, 38c, 38d), in use, faces at least one lateral surface of a superior spinous process.

4. The spacer (10) of claim 3 wherein the inner surface of one extension member of the wing (14), in use, faces a lateral surface of the superior spinous process and the inner surface of the other extension member of the wing (14) faces a lateral surface of the inferior spinous process.

5. The spacer (10) of claim 1 wherein the superior portions of the pair of extension members (38a, 38b, 38c, 38d) define a substantially U-shaped channel for the wing (14) for receiving and laterally retaining an interspinous process.

6. The spacer (10) of claim 1 wherein when in the first position, the longitudinal axis of each extension member (38a, 38b, 38c, 38d) and the longitudinal axis of the body (12) are substantially parallel.

7. The spacer (10) of claim 1 wherein when in the first position, the longitudinal axis of each extension member (38a, 38b, 38c, 38d) and the longitudinal axis of the body (12)are substantially in the same plane.

8. The spacer (10) of claim 1 wherein the first and second pair of extension members (38a, 38b, 38c, 38d) are substantially parallel.

9. The spacer (10) of claim 1 wherein the two pairs of extension members (38a, 38b, 38c, 38d) form a substantially H-shaped configuration for the wing (14).

10. The spacer (10) of claim 9 wherein, when in use in the second position, the inner surface of one extension member of the first pair faces a lateral surface of the superior spinous process and the inner surfaces of the other extension member of the first pair faces the other lateral surface of the superior spinous process and the inner surface of one extension member of the second pair faces a lateral surface of the inferior spinous process and the inner surface of the other extension member of the second pair faces the other lateral surface of the inferior spinous process.

11. The spacer (10) of claim 1 wherein the wing (14) includes a second caming surface (44).

12. The spacer (10) of claim 1 wherein the wing includes a second caming (46) surface angled with respect to the first caming surface (44).

13. The spacer (10) of claim 1 wherein the actuator (54) includes a first surface (64) for bearing against the first caming surface (44) to move the wing (14) from the first position to the second position.

14. The spacer (10) of claim 1 wherein the actuator (54) includes a first surface (64) for bearing against the first caming surface (44) for moving the wing (14)from a first position to a second configuration and a second surface (66) for bearing against the second caming surface (46) when in the second position.

15. The spacer (10) of claim 14 wherein the first surface (64) and second surface (66) of the actuator (54) define a wedge-shaped space for receiving the first caming surface and second caming surface (44, 46) of the wing (14).

16. The spacer (10) of claim 1 wherein the actuator (54) defines a wedge-shaped space for receiving the caming surface (44, 46) of the wing (14).

## Patentansprüche

1. Implantierbares Abstandsglied (10) zur Platzierung zwischen benachbarten spinösen Fortsätzen, wobei die benachbarten spinösen Fortsätze einen superioren spinösen Fortsatz und einen inferioren spinösen Fortsatz beinhalten, wobei jeder der superioren und inferioren spinöse Fortsätze zwei laterale Oberflächen aufweist, wobei das implantierbare Abstandsglied Folgendes umfasst:
einen Körper (12) mit einer Längsachse, einem ersten Ende (22) und einem zweiten Ende (20);
das erste Ende (22), das konfiguriert ist, um im interspinösen Fortsatzraum proximal zum Spinalkanal relativ zum zweiten Ende (20) positioniert zu sein;
einen Flügel (14), der mit dem Körper (12) verbunden und zur Bewegung mit Bezug auf den Körper (12) fähig ist;
den Flügel (14) mit mindestens einer Nockenoberfläche (44, 46);
eine Aktorbaugruppe (18), die mit dem Körper (12) verbunden ist;
die Aktorbaugruppe (18), die Folgendes umfasst:
einen Aktor (54); und
eine Welle (56), die mit dem Aktor (54) verbunden ist;
worin die Aktorbaugruppe (18) so konfiguriert ist, dass die Welle (56) am proximalen Ende des Abstandsglieds (20) zugänglich ist; und
worin der Aktor (54) konfiguriert ist, um sich relativ zum Körper (12) zu bewegen, um die Nockenoberfläche (44; 46) des Flügels zu berühren, um den Flügel (14) von einer ersten Position in eine zweite Position zum lateralen Stabilisieren des Abstandsglieds (10) relativ zu mindestens einem des superioren oder inferioren spinöse Fortsatzes, wenn in besagter zweiter Position, zu bewegen,
**dadurch gekennzeichnet, dass** der Körper (12) eine erste laterale Seite (24) und eine zweite laterale Seite (24) umfasst;
der Flügel (14) ein erstes Paar Verlängerungselemente (38a, 38c) mit ersten Längsachsen an der ersten lateralen Seite (24) des Körpers (12) und ein zweites Paar Verlängerungselemente (38b, 38d) mit zweiten Längsachsen an der zweiten lateralen Seite (24) des Körpers (12) umfasst;
worin der Flügel (14) zur Drehbewegung mit Bezug auf den Körper (12) fähig ist, um den Flügel (14) von der ersten in die zweite Position zu drehen;
worin die ersten Längsachsen des ersten Paares Verlängerungselemente (38a, 38c) koinzident miteinander in der ersten und zweiten Position sind und die zweiten Längsachsen des zweiten Paares Verlängerungselemente (38b, 38d) koinzident miteinander in der ersten und zweiten Position sind.

2. Abstandsglied (10) nach Anspruch 1, worin jedes Verlängerungselement (38a, 38b, 38c, 38d) eine innere Oberfläche und eine äußere Oberfläche aufweist und, wenn in der zweiten Position, in Gebrauch, jede innere Oberfläche jedes Verlängerungselements (38a, 38b, 38c, 38d) mindestens einer oder mehreren lateralen Oberflächen eines spinösen Fortsatzes zugewandt ist.

3. Abstandsglied (10) nach Anspruch 2, worin die innere Oberfläche jedes Verlängerungselements (38a, 38b, 38c, 38d), in Gebrauch, mindestens einer lateralen Oberfläche eines superioren spinösen Fortsatzes zugewandt ist.

4. Abstandsglied (10) nach Anspruch 3, worin die innere Oberfläche eines Verlängerungselements des Flügels (14), in Gebrauch, einer lateralen Oberfläche des superioren spinösen Fortsatzes zugewandt ist und die innere Oberfläche des anderen Verlängerungselements des Flügels (14) einer lateralen Oberfläche des inferioren spinösen Fortsatzes zugewandt ist.

5. Abstandsglied (10) nach Anspruch 1, worin die superioren Abschnitte des Paares Verlängerungselemente (38a, 38b, 38c, 38d) einen im Wesentlichen U-förmigen Kanal für den Flügel (14) zum Aufnehmen und lateralen Halten eines interspinösen Fortsatzes definieren.

6. Abstandsglied (10) nach Anspruch 1, worin, wenn in der ersten Position, die Längsachse jedes Verlängerungselements (38a, 38b, 38c, 38d) und die Längsachse des Körpers (12) im Wesentlichen parallel sind.

7. Abstandsglied (10) nach Anspruch 1, worin, wenn in der ersten Position, die Längsachse jedes Verlängerungselements (38a, 38b, 38c, 38d) und die Längsachse des Körpers (12) im Wesentlichen in derselben Ebene sind.

8. Abstandsglied (10) nach Anspruch 1, worin das erste und zweite Paar Verlängerungselemente (38a, 38b, 38c, 38d) im Wesentlichen parallel sind.

9. Abstandsglied (10) nach Anspruch 1, worin die zwei Paare Verlängerungselemente (38a, 38b, 38c, 38d) eine im Wesentlichen H-förmige Konfiguration für den Flügel (14) bilden.

10. Abstandsglied (10) nach Anspruch 9, worin, wenn in Gebrauch in der zweiten Position, die innere Oberfläche eines Verlängerungselements des ersten Paares einer lateralen Oberfläche des superioren spinösen Fortsatzes zugewandt ist und die innere Oberfläche des anderen Verlängerungselements des ersten Paares der anderen lateralen Oberfläche des superioren spinösen Fortsatzes zugewandt ist und die innere Oberfläche eines Verlängerungselements des zweiten Paares einer lateralen Oberfläche des inferioren spinösen Fortsatzes zugewandt ist und die innere Oberfläche des anderen Verlängerungselements des zweiten Paares der anderen lateralen Oberfläche des inferioren spinösen Fortsatzes zugewandt ist.

11. Abstandsglied (10) nach Anspruch 1, worin der Flügel (14) eine zweite Nockenoberfläche (44) beinhaltet.

12. Abstandsglied (10) nach Anspruch 1, worin der Flügel eine zweite Nockenoberfläche (46) beinhaltet, die mit Bezug auf die erste Nockenoberfläche (44) angewinkelt ist.

13. Abstandsglied (10) nach Anspruch 1, worin der Aktor (54) eine erste Oberfläche (64) zum Anliegen gegen die erste Nockenoberfläche (44) beinhaltet, um den Flügel (14) von der ersten Position in die zweite Position zu bewegen.

14. Abstandsglied (10) nach Anspruch 1, worin der Aktor (54) eine erste Oberfläche (64) zum Anliegen gegen die erste Nockenoberfläche (44) zum Bewegen des Flügels (14) von einer ersten Position in eine zweite Konfiguration und eine zweite Oberfläche (66) zum Anliegen gegen die zweite Nockenoberfläche (46), wenn in der zweiten Position, beinhaltet.

15. Abstandsglied (10) nach Anspruch 14, worin die erste Oberfläche (64) und zweite Oberfläche (66) des Aktors (54) einen keilförmigen Raum zum Aufnehmen der ersten Nockenoberfläche und zweiten Nockenoberfläche (44, 46) des Flügels (14) definieren.

16. Abstandsglied (10) nach Anspruch 1, worin der Aktor (54) einen keilförmigen Raum zum Aufnehmen der Nockenoberfläche (44, 46) des Flügels (14) definiert.

## Revendications

1. Un espaceur implantable (10) destiné à être placé entre des apophyses épineuses adjacentes, les apophyses épineuses adjacentes incluant une apophyse épineuse supérieure et une apophyse épineuse inférieure, chacune des apophyses épineuses supérieure et inférieure ayant deux surfaces latérales, l'espaceur implantable comprenant :
un corps (12) ayant un axe longitudinal, une première extrémité (22) et une deuxième extrémité (20) ;
la première extrémité (22) étant configurée pour être positionnée à l'intérieur de l'espace de l'apophyse inter-épineuse à proximité du canal rachidien par rapport à la deuxième extrémité (20) ;
une aile (14) reliée au corps (12) et pouvant effectuer un mouvement de rotation par rapport au corps (12) ;
l'aile (14) ayant une ou plusieurs surfaces de contact (44, 46) ;
un ensemble actionneur (18) relié au corps (12) ;
l'ensemble actionneur (18) comprenant :
un actionneur (54) ; et
un arbre (56) relié à l'actionneur (54) ;
l'ensemble actionneur (18) est configuré de telle sorte que l'arbre (56) est accessible à l'extrémité proximale de l'espaceur (20) ; et
l'actionneur (54) est configuré pour se déplacer par rapport au corps (12) pour entrer en contact avec la surface de contact (44 ; 460) de l'aile pour déplacer l'aile (14) d'une première position à une deuxième position afin de stabiliser latéralement l'espaceur (10) par rapport à soit l'apophyse épineuse supérieure, soit l'apophyse épineuse inférieure dans ladite deuxième position,
**caractérisé en ce que** le corps (12) comprend un premier côté latéral (24) et un deuxième côté latéral (24) ;
l'aile (14) comprend une première paire d'éléments de prolongement (38a, 38c) ayant des premiers axes longitudinaux, au premier côté latéral (24) du corps (12), et une deuxième paire d'éléments de prolongement (38b, 38d) ayant des deuxièmes axes longitudinaux, au deuxième côté latéral (24) du corps (12) ;
dans lequel l'aile (14) peut effectuer un mouvement de rotation par rapport au corps (12) pour faire tourner l'aile (14) de la première à la deuxième position ;
dans lequel les premiers axes longitudinaux de la première paire d'éléments de prolongement (38a, 38c) coïncident l'un avec l'autre dans les première et deuxième positions, et les deuxièmes axes longitudinaux de la deuxième paire d'éléments de prolongement (38b, 38d) coïncident l'un avec l'autre dans les première et deuxième positions.

2. L'espaceur (10) selon la revendication 1 dans lequel chaque élément de prolongement a (38a, 38b, 38c, 38d) une surface intérieure et une surface extérieure et, dans la deuxième position, lors de l'utilisation chaque surface intérieure de chaque élément de prolongement (38a, 38b, 38c, 38d) fait face à une ou plusieurs surfaces latérales d'une apophyse épineuse.

3. L'espaceur (10) selon la revendication 2 dans lequel la surface intérieure de chaque élément de prolongement (38a, 38b, 38c, 38d), lors de l'utilisation, fait face à une ou plusieurs surfaces latérales d'une apophyse épineuse supérieure.

4. L'espaceur (10) selon la revendication 3 dans lequel la surface intérieure d'un élément de prolongement de l'aile (14), lors de l'utilisation, fait face à une surface latérale de l'apophyse épineuse supérieure et la surface intérieure de l'autre élément de prolongement de l'aile (14) fait face à une surface latérale de l'apophyse épineuse inférieure.

5. L'espaceur (10) selon la revendication 1 dans lequel les parties supérieures de la paire d'éléments de prolongement (38a, 38b, 38c, 38d) définissent un canal sensiblement en forme de U pour l'aile (14) afin de recevoir et de retenir latéralement une apophyse inter-épineuse.

6. L'espaceur (10) selon la revendication 1 dans lequel dans la première position, l'axe longitudinal de chaque élément de prolongement (38a, 38b, 38c, 38d) et l'axe longitudinal du corps (12) sont sensiblement parallèles.

7. L'espaceur (10) selon la revendication 1 dans lequel dans la première position, l'axe longitudinal de chaque élément de prolongement (38a, 38b, 38c, 38d) et l'axe longitudinal du corps (12) sont sensiblement dans le même plan.

8. L'espaceur (10) selon la revendication 1 dans lequel les première et deuxième paires d'éléments de prolongement (38a, 38b, 38c, 38d) sont sensiblement parallèles.

9. L'espaceur (10) selon la revendication 1 dans lequel les deux paires d'éléments de prolongement (38a, 38b, 38c, 38d) forment une configuration sensiblement en forme de H pour l'aile (14).

10. L'espaceur (10) selon la revendication 9 dans lequel, lors de son utilisation dans la deuxième position, la surface intérieure d'un élément de prolongement de la première paire fait face à une surface latérale de l'apophyse épineuse supérieure et la surface intérieure de l'autre élément de prolongement de la première paire fait face à l'autre surface latérale de l'apophyse épineuse supérieure et la surface intérieure d'un élément de prolongement de la deuxième paire fait face à une surface latérale de l'apophyse épineuse inférieure et la surface intérieure de l'autre élément de prolongement de la deuxième paire fait face à l'autre surface latérale de l'apophyse épineuse inférieure.

11. L'espaceur (10) selon la revendication 1 dans lequel l'aile (14) inclut une deuxième surface de contact (44).

12. L'espaceur (10) selon la revendication 1 dans lequel l'aile inclut une deuxième surface de contact (46) inclinée par rapport à la première surface de contact (44).

13. L'espaceur (10) selon la revendication 1 dans lequel l'actionneur (54) inclut une première surface (64) pour appuyer contre la première surface de contact (44) pour déplacer l'aile (14) de la première position à la deuxième position.

14. L'espaceur (10) selon la revendication 1 dans lequel l'actionneur (54) inclut une première surface (64) pour appuyer contre la première surface de contact (44) afin de déplacer l'aile (14) d'une première position à une deuxième configuration et une deuxième surface (66) pour appuyer contre la deuxième surface de contact (46) dans la deuxième position.

15. L'espaceur (10) selon la revendication 14 dans lequel la première surface (64) et la deuxième surface (66) de l'actionneur (54) définissent un espace en forme de coin afin de recevoir la première surface de contact et la deuxième surface de contact (44, 46) de l'aile (14).

16. L'espaceur (10) selon la revendication 1 dans lequel l'actionneur (54) définit un espace en forme de coin afin de recevoir la surface de contact (44, 46) de l'aile (14).
